## Europäisches Patentamt
## European Patent Office
## Office européen des brevets

(19)

(11) Veröffentlichungsnummer: **0 065 578**
**B1**

(12)

# EUROPÄISCHE PATENTSCHRIFT

(45) Veröffentlichungstag der Patentschrift:
**13.11.85**

(51) Int. Cl.⁴: **A 61 F 9/02,** A 42 B 3/00,
G 01 D 11/30

(21) Anmeldenummer: **81103906.4**

(22) Anmeldetag: **21.05.81**

(54) **Schutzbrille bzw. Schutzhelm mit integrierter Geschwindigkeits-, Zeit- und Höhenmessung.**

(43) Veröffentlichungstag der Anmeldung:
**01.12.82 Patentblatt 82/48**

(45) Bekanntmachung des Hinweises auf die Patenterteilung:
**13.11.85 Patentblatt 85/46**

(84) Benannte Vertragsstaaten:
**AT CH DE FR LI**

(56) Entgegenhaltungen:
**DE - A - 2 619 947**
**US - A - 4 231 117**

**AVIATION WEEK AND SPACE TECHNOLOGY, Band 106, Nr. 20, Mai 1977, NEW YORK (US), B. MILLER: "Navy studies helmet-mounted sisplay", Seiten 52,53**

(73) Patentinhaber: **Teinzer, Harald, Denningerstrasse 198, D-8 München 81 (DE)**

(72) Erfinder: **Teinzer, Harald, Denningerstrasse 198, D-8 München 81 (DE)**

## Beschreibung

Die Erfindung bezieht sich auf eine Schutzbrille bzw. einen Schutzhelm.

Aus der US-amerikanischen Zeitschrift »Aviation Week and Space Technology«, Band 106 Nr. 20 (1977), Seiten 52, 53, ist ein Schutzhelm bekannt, in dem sich eine Elektronik befindet, die im Verein mit einer LED-Anzeige, einem Prisma und einer verspiegelten Sammellinse verschiedene Angaben wie Höhe, Geschwindigkeit, Beschleunigung usw. in das Blickfeld des Trägers des Schutzhelmes einspiegelt, wobei die entsprechenden Angaben von einer separaten Kontrollvorrichtung geliefert werden.

Die DE-A-2 619 947 zeigt einen Schutzhelm, der mit einem Empfänger zum Empfang von einem unabhängigen Sender ausgesendeten Signalen und mit einem von einer Batterie gespeisten optischen Signalgeber ausgerüstet ist.

Die US-A-4 231 117 betrifft einen Schutzhelm, bei dem die entsprechenden Angaben über eine Kathodenstrahlröhre und einen Reflektor Signale in das Blickfeld des Trägers eingespiegelt werden.

Aus diesen Druckschriften ist keinerlei Hinweis zu entnehmen, die einzelnen Messungen direkt an der Schutzbrille bzw. dem Schutzhelm vorzunehmen.

Die Erfindung geht vom Stand der Technik entsprechend dem Oberbegriff des Anspruchs 1 aus.

Aufgabe der Erfindung ist es — beispielsweise beim Sport wie Skifahren, Drachenfliegen — einer Person problemlos ein ständiges Überwachen der Geschwindigkeit gegenüber der Luft, der Höhe abhängig vom barometrischen Luftdruck und der Zeit relativ zu einem Startzeitpunkt zu ermöglichen.

Die Erfindung löst die Aufgabe gemäß dem Kennzeichen des Anspruchs 1. Hierbei sind an der Schutzbrille bzw. dem Schutzhelm alle Sensoren und Anzeigeelemente incl. der Auswerteelektronik für die Informationen untergebracht, die bei integrierter Geschwindigkeits-, Zeit- und Höhenmessung einer die Schutzbrille bzw. den Schutzhelm tragenden Person ständig über eine Anzeige vermittelt werden.

An der Schutzbrille bzw. dem Schutzhelm befindet sich in der Mitte von der Stirnseite ein Staurohr, in dem bei einer relativen Geschwindigkeit zur Luft ein Staudruck entsteht, der zu einem piezzoresistiven Druckaufnehmer geführt wird. Der Druckaufnehmer erzeugt ein dem Staudruck proportionales Signal, das von einer an der Brille bzw. dem Helm mitgeführten Elektronik in ein der momentanen Geschwindigkeit relativ zur Luft entsprechendes Signal umgewandelt wird. Das Signal wird digitalisiert und einer LCD-Anzeige zugeführt. Die LCD-Anzeige befindet sich über einem Auge und hat thermischen Kontakt zum Kopf. Die Darstellung auf der LCD-Anzeige erfolgt in Spiegelschrift. Die Anzeige wird über einen an der Durchsichtscheibe der Schutzbrille bzw. des Schutzhelms angebrachten Hohlspiegel oder eine verspiegelte Sammellinse für das Auge akkommodierbar in das Blickfeld eingespiegelt. Neben dem Staurohr mit Druckaufnehmer ist in der Schutzbrille bzw. dem Schutzhelm eine elektronische Stoppuhr und ein Piezzo-Absolutaufnehmer untergebracht. Über eine elektronische Fortschaltung kann wahlweise mit der LCD-Anzeige die Geschwindigkeit relativ zur Luft, die Zeit der elektronischen Stoppuhr oder die barometrische Höhe vom Absolutdruckaufnehmer abgeleitet zur Anzeige gebracht werden. Die elektronische Fortschaltung erfolgt ferngesteuert über zwei Drucktasten. Die Drucktasten befinden sich bei Einsatz der Erfindung beim Skisport am oberen Ende der beiden Skistöcke und können vom linken und rechten Daumen betätigt werden. Das Betätigen einer Drucktaste veranlaßt einen Sender, einen Sendeimpuls abzugeben, der von einem Empfänger in der Schutzbrille bzw. dem Schutzhelm empfangen wird. Der Empfänger kann unterscheiden, ob Taste 1, Taste 2 oder beide Tasten betätigt werden.

Die durch die Erfindung erreichten Vorteile sind darin zu sehen, daß z. B. während der Ausübung einer Sportart, wie Skifahren oder Drachenfliegen, problemlos die momentane Geschwindigkeit zur Luft, die Höhe abgeleitet vom barometrischen Druck oder die Zeit bezogen auf einen Startzeitpunkt visuell überwacht werden können, ohne daß die momentane Tätigkeit (z. B. Skisport) beeinträchtigt wird.

Im folgenden wird die Erfindung anhand eines Ausführungsbeispiels zum Einsatz im Skisport näher erläutert.

Die Zeichnung zeigt in Fig. 1 die Ansicht einer beim Skifahren üblichen Schutzbrille von vorne, in Fig. 2 die Ansicht der Schutzbrille von oben, in Figur die integrierte Anzeige und in Fig. 4 die oberen Enden von Skistöcken.

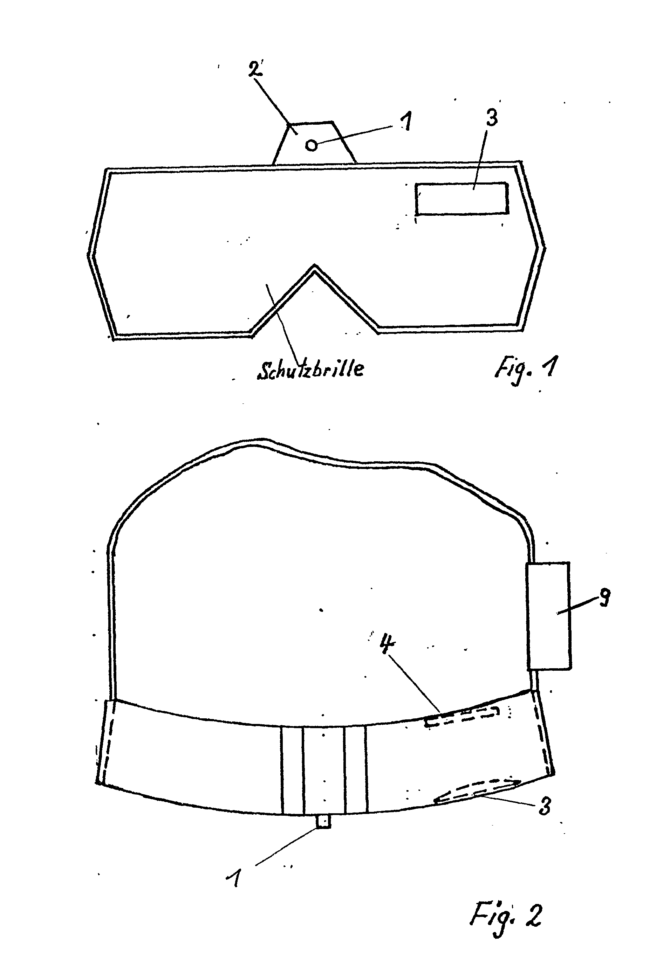

In der Mitte am oberen Rand der Schutzbrille befindet sich das Staurohr (1) mit Druckaufnehmer (2) zur Aufnahme des Luftstaudrucks (Fig. 1 und Fig. 2). Das vom Druckaufnehmer (2) erzeugte und dem Staudruck proportionale elektrische Signal wird zu einem Behälter (9) geleitet, in dem die signalverarbeitende Elektronik, Batterien, ein Absolutdruckaufnehmer, eine elektronische Stoppuhr und ein Fernsteuerempfänger untergebracht sind. Die Elektronik wandelt das vom Druckaufnehmer (2) gelieferte Signal in einen entsprechenden Geschwindigkeitswert und das vom Absolutdruckaufnehmer gelieferte Signal in einen barometrischen Höhenwert um.

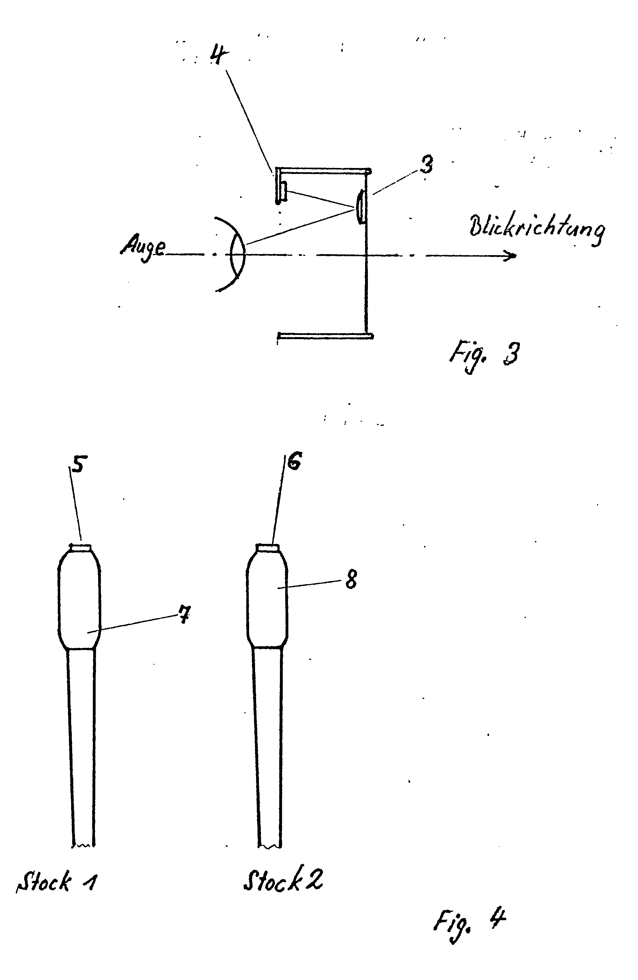

Diese beiden Werte und der von der elektronischen Stoppuhr gelieferte Wert werden auf der LCD-Anzeige (4) zur Anzeige gebracht. Die LCD-Anzeige (4) befindet sich direkt über einem Auge (Fig. 3) und hat thermischen Kontakt zur Stirn, um auch bei niedriger Umgebungstemperatur sicher zu arbeiten. Die Werte werden an der LCD-Anzeige (4) in Spiegelschrift dargestellt und entweder über eine Sammellinse mit verspiegelter Rückseite oder über einen Hohlspiegel (3) für das Auge lesbar in das Blickfeld eingespiegelt.

Die Fortschaltung der Anzeige von Zeitmessung zu Geschwindigkeits- und Höhenmessung erfolgt über die Pulstasten (5, 6, Fig. 4), die sich am oberen Ende der beiden Skistöcke befinden und vom rechten und linken Daumen betätigt werden können. Durch das Betätigen der Tasten werden in den Skistockgriffen (7 und 8) eingebaute Sender veranlaßt, Sendeimpulse abzugeben, die vom Fernsteuerempfänger im Behälter (9) empfangen werden. Im einzelnen bedeutet das Betätigen der Pulstasten folgendes:

| | |
|---|---|
| Pulstaste von Skistock 1: | Start/Stop für Zeitmessung |
| Pulstaste von Skistock 2: | Fortschaltung der Funktion |
| Pulstaste von Skistock 1 und 2: | Zurücksetzen in Ausgangslage |

## Patentansprüche

1. Schutzbrille bzw. Schutzhelm mit integrierter Geschwindigkeits-, Zeit- und Höhenmessungsanzeige, wobei die einzelnen Anzeigewerte in das Blickfeld des Trägers eingespiegelt werden, dadurch gekennzeichnet, daß als Meßeinrichtungen mit einer Auswerteelektronik ein Staudruckaufnehmer (2), bei dem der Staudruckeinlaß (1) sich in der Mitte der Kopfstirnseite befindet und der Staudruck zu einem piezzoresistiven Relativdruck-Aufnehmer geführt ist, eine elektronische Stoppuhr und ein piezzoresistiver Absolutdruck-Aufnehmer vorgesehen sind und daß als Meßwertanzeige, die mit einem Fernsteuerempfänger umschaltbar ist, eine Flüssigkristallanzeige (4), die über eine verspiegelte Sammellinse oder Hohlspiegel (3) in das Blickfeld des Trägers eingespiegelt wird, vorgesehen ist.

2. Schutzbrille bzw. Schutzhelm nach Anspruch 1, dadurch gekennzeichnet, daß die Flüssigkristallanzeige (4) sich über dem Auge befindet und thermischen Kontakt zum Kopf hat.

3. Vorrichtung zur Steuerung der Anordnung nach Anspruch 1 und 2 in der Verwendung beim Skisport, wobei die Vorrichtung aus zwei Pulstasten (5, 6) und zwei Fernsteuersendern besteht, dadurch gekennzeichnet, daß je eine Pulstaste und ein Fernsteuersender in einen Skistock eingebaut sind und die Pulstasten (5, 6) sich am oberen Ende (7, 8) des Skistocks befinden.

## Claims

1. Protective glasses or helmet with an integrated velocity, time and altitude indicator, which is reflected into the field of vision of the wearer, characterised by a measuring device and electronics comprising an air compression sensor (2) with its pressure inlet (1) arranged centrally on the forehead, which conducts the air pressure to a piezo-resistive differential pressure sensor of an electronic stop watch and of a piezo-resistive absolute pressure sensor and by a measurement indicator, being switchable via a remote control receiver comprising a LCD (4) which is introduced into the field of the wearer via a refelctive coated convex lens or concave mirror (3).

2. Protective glasses or helmet according to claim 1 characterised in that the LCD (4) is situated above the eyes and has thermal contact with the wearer's head.

3. Means for control of a device according to claims 1 and 2 for use in ski sport which consists of two pushbottons (5, 6) and two remote control transmitters, characterised in that one pushbutton and one remote transmitter are integrated into each ski stick and that the pushbutton (5, 6) is situated at the top end (7, 8) of the ski stick.

## Revendications

1. Lunettes et/ou casque de protection à affichage intégré de mesure de vitesse, de temps et d'altitude, les différentes valeurs à afficher venant se réfléchir dans le champ de vision du porteur, caractérisé(es) en ce que les dispositifs de mesure prévus, associés à une électronique d'exploitation, sont constitués d'un capteur de pression dynamique (2) dont l'entrée de pression dynamique (1) se trouve au milieu du côté frontal de la tête et dont la pression dynamique est transmise à un capteur piézorésistif de pression relative, d'un chronomètre électronique et d'un capteur piézorésistif de pression absolue, et en ce que le dispositif prévu pour l'affichage des valeurs mesurées, qui peut être commuté à l'aide d'un récepteur de télécommande, est constitué d'un affichage à cristaux liquides (4) venant se réfléchir par l'intermédiaire d'une lentille convexe métallisée ou d'un miroir concave (3) dans le champ de vision du porteur.

2. Lunettes et/ou casque de protection suivant la revendication 1, caractérisé(es) en ce que l'affichage à cristaux liquides (4) se trouve au-dessus de l'oeil et est en contact thermique avec la tête.

3. Dispositif de commande de l'agencement suivant les revendications 1 et 2 dans son application au ski, ledit dispositif étant constitué de deux touches à impulsion (5, 6) et de deux émetteurs de télécommande, caractérisé en ce qu'une touche à impulsion et un émetteur de télécommande sont incorporés dans chacun des bâtons de ski et que les touches à impulsion (5, 6) se trouvent à l'extrémité supérieure (7, 8) des bâtons.

Schutzbrille

Fig. 1

Fig. 2

4

3

Auge

Blickrichtung

Fig. 3

5

6

8

7

Stock 1

Stock 2

Fig. 4